(19) Europäisches Patentamt / European Patent Office / Office européen des brevets

(11) **EP 3 824 954 A1**

(12) **EUROPEAN PATENT APPLICATION**

(43) Date of publication:
26.05.2021 Bulletin 2021/21

(51) Int Cl.:
*A61N 5/10* (2006.01)   *A61B 18/26* (2006.01)

(21) Application number: **19306515.8**

(22) Date of filing: **22.11.2019**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br>**KH MA MD TN** | • **Université Paris-Saclay**<br>**91190 Saint-Aubin (FR)**<br>• **Institut Curie**<br>**75248 Paris (FR)**<br><br>(72) Inventor: **The designation of the inventor has not yet been filed** |
| (71) Applicants:<br>• **Centre National de la Recherche Scientifique**<br>**75016 Paris (FR)** | (74) Representative: **IPAZ**<br>**Parc Les Algorithmes, Bâtiment Platon**<br>**CS 70003 Saint-Aubin**<br>**91192 Gif-sur-Yvette Cedex (FR)** |

(54) **DEVICE, APPARATUS AND METHOD FOR MINIBEAM RADIATION THERAPY**

(57) The invention relates to a method to generate a minibeam, said method comprising the steps consisting of:
- focusing the incident beam, through a first quadrupole according to a first direction and through a second quadrupole according to a second direction orthogonal to the first direction,
- deflecting the incident beam, through a third magnet according to a third direction and through a fourth magnet according to a fourth direction different from the third direction,
- adjusting a magnetic field gradient generated by first quadrupole and/or respectively by the second quadrupole so that a focal length of the first quadrupole is superior or equal to 60 and/or is less than or equal to 250 cm and/or respectively a focal length of the second quadrupole is superior or equal to 50 and/or is less than or equal to 200 cm in order for the focused beam to meet the criteria of a minibeam along a volume extending between a focal point of the first quadrupole and a focal point of the second quadrupole.

[Fig. 1]

EP 3 824 954 A1

**Description**

**Funding**

The project leading to the results disclosed in the present invention has received funding from the European Research Council (ERC) under the European Union's Horizon 2020 research and innovation programme (grant agreement No 817908).

**Field of the invention**

The present invention concerns a method, a device and an apparatus to provide charged particle minibeam radiation therapy. The present invention relates, in particular, to a scanning nozzle and a method to generate a proton minibeam for proton minibeam radiation therapy (pMBRT).

**Background to the invention**

It is known in the art that output proton beams of a medical facility beamline, as for instance an output proton beam of a pencil beam scanning (PBS) nozzle, have narrow divergences that are usually less than 5 mrad and beam sizes of at least 4 mm full width at half maximum (FWHM). It has been found, however, that tissue sparing can be significantly improved with the pMBRT technique which requires beam sizes smaller than 2 mm FWHM which cannot be obtained with conventional PBS nozzles. It is why pMBRT techniques, carried out at clinical centers, known in the state of the art consist in emitting a proton beam from a proton beam source towards a target and arranging a collimator at a predefined distance from the target to generate an array of areas of high dose values, called peaks, adjacent to areas of low dose values, called valleys. Such minibeams significantly increase dose tolerances and sparing of normal tissue. These collimators, called multislit collimators, consist of an array of slices and slits, one slice being arranged between two slits. Such pMBRT devices are arranged to shape the proton beam and consist of a proton beam source, a nozzle and a collimator. The current nozzles are voluminous with rather long propagation distances of the beam, such that minibeam generation at clinical centers is only possible using collimators.

One drawback of using collimators lies in the reduction of the proton beam flux when crossing the collimator. In order to deliver the dose required for the pMBRT irradiation, either high beam currents at the accelerator exit are needed causing other issues or longer irradiation times are needed.

Another major drawback of using collimators lies in the significant production of harmful secondaries, or harmful side effects, close to the patient due to the proton beam being scattered by the collimator. The harmful secondaries are produced in direct vicinity of the patient and at level of the therapists while manipulating the collimator after the treatment.

Finally, the valley doses in pMBRT using collimators tends to be increased, thus a lower peak-to-valley dose ratio (PVDR) and sparing effect on healthy tissues are achieved which represents yet another disadvantage of collimators.

**Summary of the invention**

An object of the invention is to provide a minibeam scanning nozzle and a method to generate charged particle minibeams. Such a minibeam scanning nozzle:

- addresses at least some of the drawbacks of the state of the art, and/or
- is suitable to be mounted on current charged particle radiation therapy (RT) equipment and is arranged not to produce harmful secondaries produced in the direct vicinity of the patient, and/or
- is suitable to be mounted on current charged particle RT equipment and is arranged to decrease flux losses of the proton beam upstream to the patient, and/or
- is suitable to be mounted on current charged particle RT equipment and is arranged to increase the PVDR, and/or
- has smaller dimensions than current nozzles, and/or
- provides charged particle minibeams from an incident beam of charged particles, and/or
- eliminates the need for using a collimator.

According to the present invention, there is provided a method to generate a minibeam, said method comprising the steps consisting of:

- focusing the incident beam, through a first quadrupole according to a first direction,
- focusing the incident beam, through a second quadrupole according to a second direction orthogonal to the first direction,

- deflecting the incident beam, through a third magnet according to a third direction,
- deflecting the incident beam through a fourth magnet according to a fourth direction different from the third direction,
- adjusting a magnetic field gradient generated by the first quadrupole and/or respectively by the second quadrupole so that a focal length of the first quadrupole is superior or equal to 60 and/or is less than or equal to 250 cm and/or respectively a focal length of the second quadrupole is superior or equal to 50 and/or is less than or equal to 240 cm in order for the focused beam to meet the criteria of a minibeam along a volume extending between a focal point of the first quadrupole and a focal point of the second quadrupole.

Preferably, the fourth direction is orthogonal to the third direction.

According to the method, the minibeam may be generated from an incident beam of charged particles that exhibits:

- an energy superior or equal to 10 and/or less than or equal to 1000 MeV, and
- a divergence less than 15 milliradian, and/or
- an absolute value of a correlation coefficient between a size of the incident beam and the divergence of the incident beam superior or equal to 0.8 and/or less than or equal to 1.

Preferably, the minibeam generated through the method according to the invention is intended to be used for minibeam radiation therapy.

According to the invention, the term adjust may be understood as set.

According to the invention, the magnetic field gradients may be determined, set or adjusted based on the parameters of the incident beam, for instance the energy and/or the divergence and/or the size and/or an absolute value of a correlation coefficient between a size of the incident beam and the divergence of the incident beam.

The minibeam may be generated from an incident beam of charged particles that exhibits:

- an energy superior or equal to 10 and/or less than or equal to 1000 MeV, and
- a divergence less than 1 milliradian, and
- an absolute value of a correlation coefficient between a size of the incident beam and the divergence of the incident beam superior or equal to 0.8 and/or less than or equal to 1.

The minibeam may be generated from an incident beam of charged particles that exhibits:

- an energy superior or equal to 10 and/or less than or equal to 1000 MeV, and
- a divergence less than 15 milliradian, and
- an absolute value of a correlation coefficient between a size of the incident beam and the divergence of the incident beam superior or equal to 0.95 and/or less than or equal to 1. Preferably, the absolute value of the correlation coefficient of the incident beam is equal to 1.

Optionally, the energy of a particle of the incident beam of charged particles is less than 1000 MeV/nucleon.

Optionally, the energy of a particle of the incident beam of charged particles is comprised between 50 and 250 MeV, preferably between 100 and 230 MeV

The higher the divergence of the incident beam becomes, the higher the absolute value of a correlation coefficient between a size of the incident beam and the divergence of the incident beam may be. The lower the absolute value of a correlation coefficient between a size of the incident beam and the divergence of the incident beam becomes, the lower the divergence of the incident beam may be.

The size of the incident beam may be defined as a diameter of the incident beam. If the incident beam is considered as a Gaussian beam, the size of the beam may be defined through a couple consisting of a vertical full width at half maximum (vFWHM) and a horizontal FWHM (hFWHM).

A focal length of the second quadrupole, or respectively of the first quadrupole, may be defined as the distance between the second quadrupole, or respectively the first quadrupole, and a focal point of the second quadrupole, or respectively of the first quadrupole.

A distance between a focal point of the first quadrupole and a focal point of the second quadrupole may be less than or equal to 50 cm in order for the focused beam to meet the criteria of a minibeam along the volume extending between the focal point of the first quadrupole and the focal point of the second quadrupole.

Preferably, the focal length of the first quadrupole and/or the focal length of the second quadrupole is superior or equal to 60 cm, more preferably superior or equal to 70 cm, even more preferably superior or equal to 80 cm and in a preferred manner superior or equal to 90 cm.

Preferably, the focal length of the second quadrupole is less than or equal to 180 cm, more preferably less than or equal to 160 cm, even more preferably less than or equal to 150 cm, in a preferred manner less than or equal to 140 cm, in a more preferred manner less than or equal to 130 cm and in a particularly preferred manner less than or equal to 120 cm.

Preferably, the focal length of the first quadrupole is less than or equal to 220 cm, more preferably less than or equal to 200 cm, even more preferably less than or equal to 180 cm, in a preferred manner less than or equal to 160 cm, in a more preferred manner less than or equal to 140 cm, in a particularly preferred manner less than or equal to 130 cm.

The adjustment of the magnetic field gradient generated by the second quadrupole and/or of the magnetic field gradient generated by the first quadrupole may be defined as controlling the magnetic field gradients generated by the second quadrupole and/or by the first quadrupole so that the vFWHM and hFWHM of the focused beam meet the criteria of a minibeam. Preferably, the vFWHM and hFWHM of the focused beam meet the criteria of a minibeam in the volume extending between the focal point of the first quadrupole and the focal point of the second quadrupole.

The incident beam may be deflected through the third and fourth magnets so as to move a point of intersection between the charged particle minibeam and a target.

The first and second quadrupoles and the third and fourth magnets may be part of a nozzle. The nozzle may be a scanning nozzle. The nozzle may be a scanning nozzle according to the invention.

Preferably, downstream of the exit of the scanning nozzle, according to a beam path, the focused beam is focused and deflected.

The method may comprise the step consisting of arranging the beam, the first and second quadrupoles and the third and fourth magnets in a volume, where the beam is contained in a vacuum environment, said vacuum environment extending over a distance higher than 50 cm and lower than 200 cm.

Preferably, the magnets are not in the vacuum chamber, only the beam. The vacuum chamber or tube passes through the magnets. It is however possible to design a system where all components are in a vacuum environment, but only an ionization chamber can be in an air filled environment.

The vacuum environment may be generated inside a vacuum chamber. The vacuum chamber may be a vacuum chamber of a scanning nozzle.

A distance from an end of the vacuum environment to the focal point of the second quadrupole may be superior to few centimeters and/or is less than or equal to 50 cm.

Preferably, a distance from an end of the vacuum environment to the focal point of the second quadrupole may be superior or equal to 10 cm.

Preferably, a distance from an end of the vacuum environment to the focal point of the second quadrupole may less than or equal to 40 cm, more preferably less than or equal to 30 cm.

The end of the vacuum environment may be an edge of the vacuum environment and/or an end of the vacuum chamber and/or an edge of the vacuum chamber.

A FWHM of the incident beam may be less than 50 mm.

Values of magnetic field gradients generated by the first and second quadrupoles, required so that the beam focused at the focal point of the second quadrupole meets the criteria of a minibeam, may be superior or equal to 0 and/or are less than or equal to $1.6 \text{ T.cm}^{-1}$.

A distance separating the first quadrupole from the second quadrupole may be less than 15 cm, preferably less than 6 cm, more preferably less than or equal to 3 cm.

The incident beam of charged particles may exit from a beamline of a medical facility. In other words, the incident beam of charged particles may be an output beam of a beamline of a medical facility

The charged particles may be ions. Preferably, the charged particles are protons or carbon ions.

Operational frequencies of the third and fourth magnets may be superior or equal to 1 Hz and/or are less than or equal to 200 Hz.

According to the invention, a minibeam is defined as the maximum size of a beam under which the desired tissue sparing effect takes place. This maximum beam size is defined as being a full width at half maximum (FWHM) value of the beam equal to 2 mm at an entrance plane of a target to be irradiated. By analogy, this maximum beam size may also be defined as being equal to $2.355\,\sigma$ of the beam Gaussian distribution, where $\sigma$ is the standard deviation. The entrance plane of a target to be irradiated may be located in the volume extending between the focal point of the first quadrupole and the focal point of the second quadrupole.

Preferably, according to the invention, the minibeam is defined as a beam that exhibits a FWHM less than or equal to 2 mm.

A minibeam according to the invention may exhibit a horizontal full width at half maximum (hFWHM) less than or equal to 2 mm and a vertical FWHM (vFWHM) equal to or less than the hFWHM of the minibeam. In other words, a minibeam according to the invention may exhibit a FWHM less than or equal to 2 mm.

Preferably, according to the invention, a minibeam exhibits a FWHM, at the volume extending between the focal point of the first quadrupole and the focal point of the second quadrupole, less than or equal to 1 mm, more preferably less than or equal to 0.9 mm, in a preferred manner less than or equal to 0.8 mm and in a more preferred manner less than or equal to 0.7 mm.

The method may comprise the step consisting of measuring the intensity and/or the spatial location of the focused beam downstream of the first and second quadrupoles and the third and fourth magnets according to the beam path. Preferably, the method may comprise the step consisting of measuring the intensity and/or the spatial location of the focused beam at a position located between the fourth magnet and the focal point of the first quadrupole.

According to the present invention, there is also provided a minibeam scanning nozzle (MSN) for charged particle minibeam radiation therapy, said MSN comprising, along a beam path of the charged particles inside the nozzle:

- a first quadrupole arranged to focus the incident beam according to a first direction,
- a second quadrupole arranged to focus the incident beam according to a second direction orthogonal to the first direction,
- a third magnet arranged to deflect the incident beam according to a third direction, and
- a fourth magnet arranged to deflect the incident beam according to a fourth direction different from the third direction;

the first quadrupole and/or respectively the second quadrupole being arranged to generate a magnetic field gradient that is adjusted so that a focal length of the first quadrupole is superior or equal to 60 and/or is less than or equal to 250 cm and/or respectively a focal length of the second quadrupole is superior or equal to 50 and/or is less than or equal to 200 cm in order for the focused beam to meet the criteria of a minibeam along a volume extending between a focal point of the first quadrupole and a focal point of the second quadrupole.

The MSN may comprise a vacuum chamber wherein the beam is contained passing through the first and second quadrupoles and the third and fourth magnets. The vacuum chamber is preferably arranged in the bore of the magnets.

A distance between an exit face of the vacuum chamber and the focal point of the second quadrupole may be less than 50 cm.

The MSN may be arranged to generate the minibeam from an incident beam of charged particles exiting a beamline of a medical facility, the MSN being intended to be arranged downstream of the beamline in a path of the incident beam of charged particles.

The third and/or the fourth magnets may be scanning dipole magnets.

Optionally, the first quadrupole and/or the second quadrupole and/or the third magnet and/or the fourth magnet are superconducting magnets.

Optionally, the first and/or the second quadrupoles are arranged to generate magnetic field gradient superior or equal to 0 and/or less than or equal to 1.6 T.cm$^{-1}$.

Optionally, the first, second, third and/or fourth magnets are part of a multipole magnet. The multipole magnet may comprise eight or more poles.

Optionally, the first, second, third and/or fourth magnets are part of an octupole magnet.

The third and/or the fourth magnets may be arranged to deflect the beam propagating within the vacuum chamber so as to move a point of intersection between the charged particles minibeam and the target.

The scanning nozzle may comprise one or more ionization chambers.

Optionally, a gaseous material filling the ionization chambers is an inert gas or a noble gas.

Preferably, the gaseous material filling the ionization chambers is air or helium or a gas mixture.

The MSN according to the invention may be arranged to carry out the method according to the invention.

Use of the MSN according to the invention for implementing the method according to the invention.

Optionally, the MSN may be used for irradiating a tumor. Optionally, the MSN may be used for the treatment of a tumor located in a patient. Optionally, the MSN may be used for the treatment of cancer.

According to the present invention, there is also provided a system for charged particles minibeam radiation therapy comprising:

- a minibeam scanning nozzle (MSN) arranged to generate a minibeam of charged particles comprising, along a beam path of the charged particles inside the nozzle:

  - a first quadrupole arranged to focus the incident beam according to a first direction,
  - a second quadrupole arranged to focus the incident beam according to a second direction orthogonal to the first direction,
  - a third magnet arranged to deflect the incident beam according to a third direction, and
  - a fourth magnet arranged to deflect the incident beam according to a fourth direction different from the third direction;

the first quadrupole and/or respectively the second quadrupole being arranged to generate a magnetic field gradient that is adjusted so that a focal length of the first quadrupole is superior or equal to 60 and/or is less than or equal to 250 cm and/or respectively a focal length of the second quadrupole is superior or equal to 50 and/or is less than or equal to 240 cm in order for the focused beam to meet the criteria of a minibeam along a volume extending between a focal point of the first quadrupole and a focal point of the second quadrupole.

The system for charged particles minibeam radiation therapy may comprise a beam source arranged to generate an incident beam of charged particles that exhibits:

- an energy superior or equal to 10 and/or less than or equal to 1000 MeV, and
- a divergence less than 15 milliradian, and/or
- an absolute value of a correlation coefficient between a size of the incident beam and the divergence of the incident beam superior or equal to 0.8 and/or less than or equal to 1.

The beam source may comprise a beamline of a medical facility.

The MSN of the system for charged particles minibeam radiation therapy may be the MSN according to the invention.

**Brief description of the drawings**

Further inventive objects, features and advantages will become apparent from the following detailed description of several embodiments of the invention with references to the drawings, in which:

- FIGURE 1 is a schematic tilted view of an arrangement of the nozzle components according to the invention,
- FIGURE 2a is a schematic side view of a setup currently used in medical facilities for radiation therapy,
- FIGURES 2b, 2c and 2d are schematic side view of three further possible arrangements of a setup for radiation therapy,
- FIGURE 3a, 3b, 3c and 3d show four bar charts illustrating the minimized sizes of the focused beam achieved respectively by the nozzle arrangements of Figures 2a, 2b, 2c and 2d.

**Detailed description of embodiments of the invention**

The embodiments hereinafter described are not restrictive; other embodiments comprising a selection of features described hereinafter may be considered. A selection may comprise features isolated from a set of features (even if this selection is isolated among a sentence comprising other features thereof), if the selection is sufficient to confer a technical advantage or to distinguish the invention form the state of the art. This selection comprises at least a feature, preferably described by its technical function without structural features, or with a part of structural details if this part is sufficient to confer a technical advantage or to distinguish the invention form the state of the art on its own.

An embodiment of a method to generate a minibeam according to the invention is described. This method is intended to be used for minibeam radiation therapy. The method comprises the steps consisting of:

- focusing the incident beam 6, through a first quadrupole 10 according to a first direction x,
- focusing the incident beam 6, through a second quadrupole 11 according to a second direction y orthogonal to the first direction x,
- deflecting the incident beam 6, through a third magnet 12 according to a third direction y,
- deflecting the incident beam 6, through a fourth magnet according to a fourth direction x orthogonal to the third direction y,
- adjusting a magnetic field gradient generated by the first quadrupole 10 and/or respectively by the second quadrupole 11 so that a focal length of the first quadrupole 10 is superior or equal to 60 cm and/or is less than or equal to 250 cm and/or respectively a focal length of the of the second quadrupole 11 is superior or equal to 50 cm and/or is less than or equal to 240 cm in order for the focused beam 16 to meet the criteria of a minibeam along a volume extending between a focal point of the first quadrupole 10 and a focal point of the second quadrupole 11.

In practice, the magnetic field gradient generated by the first quadrupole 10 can be predetermined or set, for example, so that the target 7 (for example a tumor 7), or a section plane 18 of the target 7 or an entrance plane 18 of the target 7, is arranged at a given distance of an exit plane 33 of the nozzle 2 according to the invention. The magnetic field gradient generated by the second quadrupole 11 is then adjusted, according to the invention, so that the focused beam 16 meets the criteria of a minibeam at the target 7 or at a section plane 18 of the target 7 or at an entrance plane 18 of the target 7. Conversely, the magnetic field gradient generated by the second quadrupole 11 can be predetermined or set, for example, so that the target 7 or a section plane 18 of the target 7 or an entrance plane 18 of the target 7, is arranged at a given distance of an exit plane 33 of the nozzle 2 according to the invention.

EP 3 824 954 A1

**EP 3 824 954 A1**

Usual pMBRT system comprises a beam source. The beam source comprises a proton accelerator, such as a cyclotron, and a beam transport system comprising magnets and being arranged to convey an incident proton beam 6 from the proton accelerator to the nozzle 2. For other therapies, protons may be substituted with other ions, such as carbon ions. After the transport system, the beam is finally shaped with a collimator to generate an array of areas of high dose values and/or is deflected to generate a scanning beam.

An embodiment according to the invention comprises a proton minibeam radiation therapy (pMBRT) system (not represented). The system comprises a nozzle 2 according to the invention. The nozzle 2 according to the invention is arranged to shape the incident proton beam 6 into a proton minibeam 16 and to guide said proton minibeam 16 towards a tumor (not depicted) located in a patient 7.

In some pMBRT system, the patient is located in a gantry which is part of the pMBRT system. The nozzle 2 is connected to the gantry (not represented) and the gantry is arranged to rotate the nozzle 2 around the patient 7 so as to enable treatment with multiple fields from different angles to better target the tumor and spare surrounding healthy tissue.

Typical $\sigma$ values of current proton beams 6 used in current facilities are between 2 and 10 mm. Standard energy values of current proton beams 6 used in current facilities are between 60 and 230 MeV. A standard divergence exhibited by current proton beams 6 is around 3 mrad (milliradians).

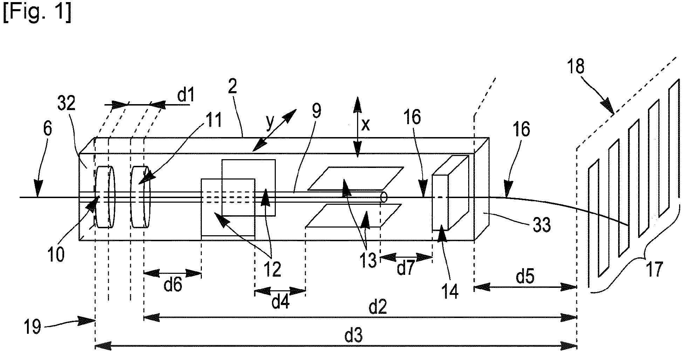

FIGURE 1 shows an embodiment of the nozzle 2 arrangement according to the invention that has been found suitable for minibeam generation compared to numerous different potential nozzle arrangements investigated (see below) that are not suitable. The nozzle 2 comprises a vacuum chamber 9 arranged to receive the incident proton beam 6. Coming from the transport system, the proton beam propagates into the vacuum chamber 9. According to the beam path, in other words from left to right of the figure 1, the nozzle 2 comprises:

- a first quadrupole 10,
- a second quadrupole 11,
- a first scanning dipole 12, and
- a second scanning dipole 13.

In a preferable configuration, the nozzle 2 also comprises an ionization chamber 14.

The vacuum chamber 9 extends from an entrance face 32 of the nozzle 2, through which the proton beam 6 enters the vacuum chamber 9, to an exit of the dipole 13, through which the proton minibeam 16 exits the vacuum chamber 9 towards the target 7 (which can be a tumor for example) through an ionization chamber 14. The quadrupoles 10, 11, the dipoles 12, 13 are out of the vacuum environment of the vacuum chamber 9. The proton beam propagates in the vacuum chamber through each quadrupole 10, 11, then between the two poles of each scanning dipole 12, 13 and is located at equal distance from each pole of a scanning dipole 12, 13. Then, the proton minibeam 16 passes through the ionization chamber 14 before exiting the vacuum chamber and propagating towards the target 7.

The first quadrupole 10 is arranged to focus the proton beam 6 propagating within the vacuum chamber 9 according to the y direction and the second quadrupole 11 is arranged to focus the proton beam 6 propagating within the vacuum chamber 9 according to the x direction. Each of the first 10 and the second quadrupoles 11 is arranged to provide a variable magnetic field gradient. Downstream of the second quadrupole 11, according to the beam path, the proton beam is focused to meet the criteria of a minibeam along a volume extending between a focal point of the first quadrupole 10 and a focal point of the second quadrupole 11.

The ionization chamber 14 is arranged to measure the intensity and the size and position of the proton minibeam 16. The wall parts of each ionization chamber located on the beam path may be made of mylar. The ionization chamber 14 is filled with air or helium.

The first scanning dipole 12 is arranged to deflect the proton beam propagating within the vacuum chamber 9 according to the y direction and the second scanning dipole 13 is arranged to deflect the proton beam 6 propagating within the vacuum chamber 9 according to the x direction. Each of the first 12 and the second scanning dipole 13 is arranged to provide an approximatively homogeneous magnetic field of variable strength. A processing unit, for use in connection with the nozzle 2, is arranged to control the magnetic field gradient value provided by each of the first 12 and the second scanning dipole 13 so as to deflect the proton beam 6 propagating within the vacuum chamber 9 so as to move the point of intersection between the proton minibeam 16 and a given section plane 18 of the target 7. The point of intersection between the proton minibeam 16 and the section plane 18 of the target 7 is moved so as to scan the target 7 according to a given pattern 17. According to the embodiment, the pattern 17 exhibits a crenellated shape. The pattern may also be any type of alternating array of areas of high dose values, called peaks and areas of low dose values, called valleys. As non-limiting examples, the areas may be circles or ellipses or squares or rectangles and may be concentric. Each of the first 12 and the second 13 scanning dipole exhibits an operational frequency between 1 and 200 Hz. An operational frequency range of 3 to 100 Hz is suitable in most cases. Downstream of the second scanning dipole 13, according to the beam path, the proton beam 16 is focused and deflected.

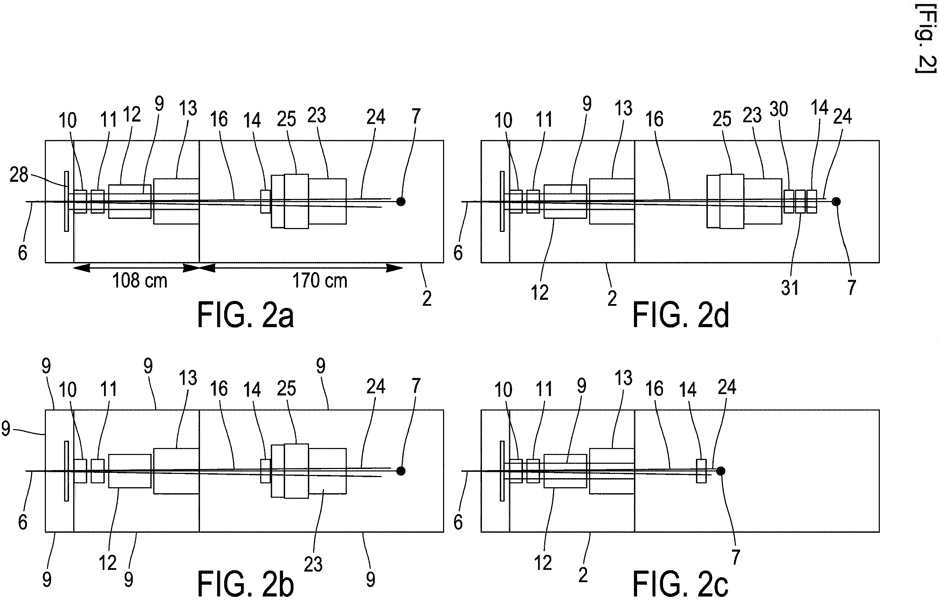

Embodiments on figures 2a, 2c and 2d illustrate a chamber 2 containing a vacuum chamber 9 conveying the beam. The vacuum chamber passes through the components 10-13. The magnets are disposed around the vacuum chamber, not inside. Others components are in an air filled volume. On figure 2b, the whole chamber 2 is in a vacuum environment, thus it is considered as the vacuum chamber 9. In all figures, including figure 2b, the ionization chamber 14 is always arranged in an air filled volume.

FIGURE 2a illustrates a setup currently used in medical facilities for radiation therapy. The current setup comprises a snout 23 arranged to receive a collimator and to obtain a shaped proton beam 24 from the focused beam 16, which is not a minibeam, prior to impact the target 7. The snout 23 is in the ambient air and is mounted on a snout holder 25. A ionization chamber 14 is placed downstream of the vacuum chamber 9. The current setup comprises a vacuum window 28 arranged in the ambient air upstream a vacuum chamber 9. A first 10 and a second 11 quadrupole and a first 12 and a second 13 scanning magnet are arranged out of the vacuum chamber 9.

A substantial number of different modifications of the current setup, as described above and as illustrated Figure 2a, have been tested. These modifications have led to numerous different potential arrangements of the setup. Among these numerous different potential arrangements, four are set forth in Figures 2b, 2c and 2d. Besides the current setup, shown in Figure 2a, each of these four arrangements has been investigated as described hereinafter. The modifications carried out comprise, among others, the arrangement of the elements relative to each other and/or the distance between the elements and the target and/or the removal of the snout 23, the snout holder 25 and the ionization chamber 14 and/or the addition of extra elements at different locations of the setup, in particular a pair of extra quadrupoles 30, 31.

Simulations of proton beams propagating through nozzles were performed. The simulations were Monte Carlo simulations performed carried out with TOPAS version 3.2 and 3.1. For each given setup arrangement investigated, energies of the incident proton beam of 100, 150 and 200 MeV were considered and the magnetic field gradient in each of the quadrupoles 10, 11 and was varied in steps of 0.016 T.cm$^{-1}$ from 0 to 0.8 T.cm$^{-1}$, resulting in 51 different field strengths for each quadrupole 10, 11. Furthermore, two orientations of the focussing planes of the quadrupoles 10, 11 were considered. The minimum beam size for each setup arrangement was then determined by comparing the sizes simulated with each of these 51 x 51 x 2 configurations of the quadrupoles 10, 11. It has to be noted that the magnetic field applied at the pole tips generates a magnetic field gradient inside the quadrupole 10, 11.

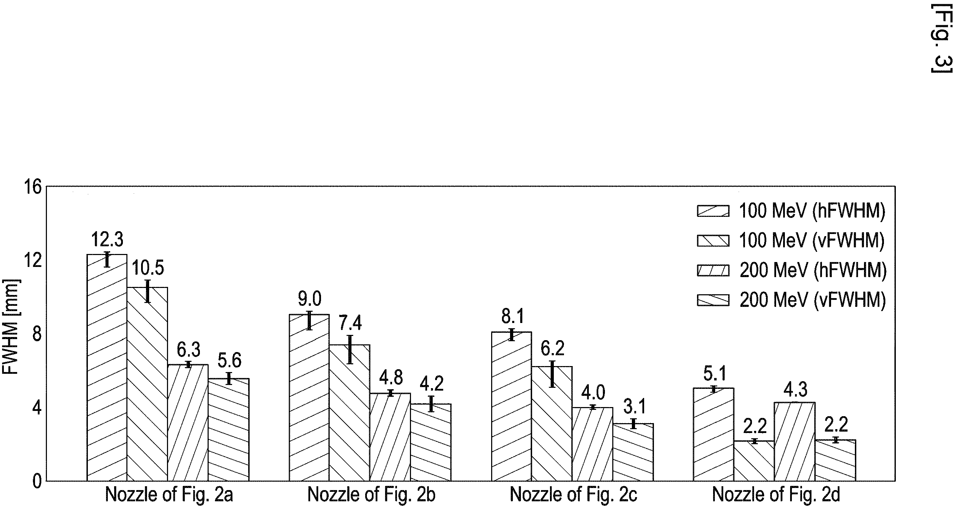

Figure 3 shows four bar charts depicting the minimum sizes of the focused beam 16, calculated from the simulations, achieved by the setup arrangements of Figures 2a, 2b, 2c and 2d from left to right. The bar charts of Figures 3a, 3b and 3d related to arrangements of Figures 2a, 2b and 2d show the horizontal FWHM (hFWHM) and the vertical FWHM (vFWHM) of the shaped proton beam 24 at the target 7 for an energy of the incident proton beam 6 of 100 and 200 MeV and the chart of the Figure 3c related to the arrangement of Figure 2c shows the horizontal FWHM (hFWHM) and the vertical FWHM (vFWHM) of the focused beam 16 at the target 7 for energy of the incident proton beam 6 of 100 and 200 MeV. None of the presented arrangements is suitable to provide minibeam, that is to say a beam that exhibits a hWHM and a vFWHM less than 2 mm. Among the numerous different potential arrangements investigated, only the arrangement of the setup according to the invention, set forth Figure 1, allows to achieve minibeams. The minimized beam sizes of the nozzle 2 according to the invention, set forth in Figure 1, derived from the simulations, are presented in chart 1.

Hence, compared to usual pencil beam scanning (PBS) of Figure 2a that produces beams with minimum FWHM of 5 mm at the target 7, the beam 16 produced by the nozzle 2 according to the invention meets the criteria of a minibeam at a target 7 and may be used for pMBRT.

Chart 1

| Configuration | | Beam size at the target | |
|---|---|---|---|
| Incident beam energy (MeV) | Distance between the nozzle exit and the target (cm) | hFWHM (mm) | vFWHM (mm) |
| 100 | 30 | 1.67 | 1.65 |
| 100 | 10 | 0.66 | 0.68 |
| 200 | 30 | 0.87 | 0.85 |
| 200 | 10 | 0.33 | 0.35 |

Throughout the rest of the description, the nozzle 2 according to the invention, as set forth in Figure 3, is considered. The precise dimensions of the nozzle 2 and the features of the nozzle 2 components, the distances separating the components of the nozzle 2 between each other and the components of the nozzle 2 from the target 7 have been simulated with an analytical calculation code for magnetic fields and checked through Monte Carlo method using TOPAS code version 3.2 and 3.1. The components comprise the first 10 and the second 11 quadrupoles, the third 12 and the fourth 13 scanning dipoles and the ionization chamber 14.

The size of each component of the nozzle 2, the distances separating these components from one another and the distances separating these components from the target 7 are introduced. Each component length presented hereinafter describes the size of a component along the beam path. The size of each component is defined by the distance separating an entrance plane and an exit plane of a component.

It has been found that one of the major parameters, related to the nozzle 2 arrangement, to be controlled to achieve a minibeam is the distance d2 separating the exit face of the second quadrupoles 11 from the section plane 18 of the target 7. To obtain a minimum size of the focused beam 16, the distance d2 should be less than 200 cm. A distance d2 less than 200 cm is only achievable by means of the nozzle 2 arrangement according to the invention and also because the nozzle 2 according to the invention does not use a snout 23 or a physical collimator. Indeed, the distance d2 in current setups used in medical facilities, as shown Figure 2a, is generally superior to 230 cm.

It has been observed that another relevant parameter to be controlled to achieve a minibeam is the distance (d5) between the exit plane 33 of the vacuum tank 2 and the section plane 18 of the target 7. From the calculation, to obtain minimum sizes of the focused beam 16, this distance d5 is less than 50 cm. Here again, such a distance d5 is achievable because the nozzle 2 according to the invention does not use a snout 23 or a physical collimator.

In the state of the art, the minibeams, and in particular the minibeams generated from medical facilities, are considered to be achievable only by using a collimator mounted on the snout 23 and/or a collimator. Therefore, removing the snout 23 and/or the collimator has been counterintuitive.

In reference to Figure 1, the distance between the entrance plane 32 of the nozzle 2 and the entrance face of the first quadrupole 10, the first quadrupoles 10 length (lq1) and the second quadrupoles 11 length (lq2), the distance d1 separating the first quadrupoles 10 and the second quadrupoles 11, the first scanning dipole 12 length (ld1), the second scanning dipole 13 length (ld2), the distance between the exit face of the second quadrupole 11 and the entrance face of the first scanning dipole 12 (d6), the distance between the exit face of the first scanning dipole 12 and the entrance face of the second scanning dipole 13 (d4), the distance between the exit face of the second scanning dipole 13 and the entrance face of the ionization chamber 14 (d7), the ionization chamber 14 length (li1), the distance between the exit face of the ionization chamber 14 and the exit plane 33 of the nozzle 2 and the distance d5 between the exit plane 33 of the nozzle 2 and the section plane 18 of the tumor are adapted so that d2 and d5 fit with the values described hereinabove.

The following example is a particular embodiment arrangement of the nozzle 2 and its component relative to the target 7. This is a preferred embodiment among numerous embodiments which is given for the purpose of further explanation and not limitation. A quadrupole 10, 11 usually has a cylindrical shape. From the calculation, the diameter of this assembly is 20 cm in the present embodiment; the diameter being orthogonal to the beam path. A height and a width of the dipoles 12, 13 and the ionization chamber 9 are 20 cm. A diameter of the vacuum chamber 9 is about 5 cm. A vacuum is maintained within the nozzle 2. In this preferred embodiment, The distance lq1 is equal to lq2 is less than 20 cm, preferably comprised between 3 and 20 cm, more preferably comprised between 5 and 15 cm and is equal to 10 cm in the present embodiment. The distance d1 is less than 15 cm, preferably less than 6 cm and is equal to 3 cm in the present embodiment. The distance li1 is less than 30 cm, preferably comprised between 2 and 15 cm, and is equal to 10 cm in the present embodiment. The distance d2 is less than 200 cm, and preferably between 90 and 110 cm in the present embodiment. The distance (d3) between the beam entrance face 32 the nozzle 2 and the section plane 18 of the tumor is less than 260 cm, generally comprised between 130 and 160 cm and preferably between 130 and 150 cm in the present embodiment. The distance ld1 is equal to ld2 is less than 40 cm and is equal to 25 cm in the present embodiment. The distance d4 is less than 10 cm and is equal to 0 cm in the present embodiment. The distance d6 is less than 15 cm and is equal to 5 cm in the present embodiment. The distance d7 is less than 30 cm and is equal 15 cm in the present embodiment.

The nozzle 2 according to the invention avoids the use of collimators. The use of collimators in current pMBRT devices causes the loss of a major part of the total flux of the proton beam 6. Thus, the dose rate is strongly lowered. The nozzle 2 according to the invention allows transmitting the total flux of the proton beam 6 to the tumor. Thus, the dose rate is significantly increased compared to current pMBRT. Moreover, avoiding the use of a collimator allows to significantly decrease the production of harmful secondaries close to the patient.

In order for the focal length of the second quadrupoles 11 to be less than 200 cm, and so for the FWHM of the focused beam 16 to meet the criteria of a minibeam, the magnetic field gradient to be generated has been investigated. To that end, it has been found that minimizing $\Omega$ was a pertinent approach, where

$$\Omega = (\text{hFWHM})^2 + (\text{vFWHM})^2.$$

The calculations have been carried out for the 51 x 51 x 2 quadrupole 10, 11 configurations and with magnetic field gradients defined by the magnetic field applied at the pole tips of the first 10 and the second 11 quadrupoles which was incremented in steps of 0.016 T.cm$^{-1}$ from 0 to 0.8 T.cm$^{-1}$.

In order for the focused beam 16 to meet the criteria of a minibeam along a volume extending between a focal point of the first quadrupole 10 and a focal point of the second 11 quadrupole, the magnetic field gradient generated by the first 10 and/or the second 11 quadrupoles are adjusted, by the processing unit, so that the focal length of the first 10 quadrupole is superior or equal to 60 and/or is less than or equal to 250 cm and/or the focal length of the second 11 quadrupole is superior or equal to 50 and/or is less than or equal to 240 cm. According to the embodiment, the magnetic field gradients generated by the quadrupoles 10, 11 are adjusted so that:

- the focal point of the first quadrupole 10 tends superior or equal to 108 cm and the focal point of the second 11 quadrupole is inferior or equal to 128 cm, or
- the focal point of the first quadrupole 10 is inferior or equal to 128 cm and the focal point of the second 11 quadrupole is inferior or equal to 108 cm. Hence, the target 7, or a section plane 18 of the target 7 or an entrance plane 18 of the target 7, will be positioned at an optimal distance, which is between 10 and 30 cm, from the exit plane 33 of the nozzle 2.

The inventors showed that the focusing capabilities of the nozzles strongly depend on parameters of the incident beam 6. In particular, the inventors observed that at least one among the divergence of the incident beam 6 and the correlation coefficient between the size of the incident beam 6 and the divergence of the incident beam 6 has to be controlled. Counterintuitively, the size of the incident beam 6 has a negligible effect.

From the calculations, in order for the FWHM of the focused beam 16 to meet the criteria of a minibeam, it has been found that:

- either the divergence of the incident beam 6 is less than 15 milliradian, and/or
- the absolute value of the correlation coefficient between a size of the incident beam 6 and the divergence of the incident beam 6 is superior or equal to 0.8 and/or less than or equal to 1.

The combination of a divergence of the incident beam 6 less than 15 milliradian and an absolute value of the correlation coefficient between a size of the incident beam 6 and the divergence of the incident beam superior or equal to 0.95 and/or less than or equal to 1 will ensure the focused beam 16 to meet the criteria of a minibeam. Conversely, the combination of a divergence of the incident beam 6 less than 1 milliradian and an absolute value of the correlation coefficient between a size of the incident beam 6 and the divergence of the incident beam superior or equal to 0.9 and/or less than or equal to 1 will ensure the focused beam 16 to meet the criteria of a minibeam.

Indeed, the combination of a divergence of the incident beam 6 less than 1 milliradian and an absolute value of the correlation coefficient between a size of the incident beam 6 and the divergence of the incident beam superior or equal to 0.95 and/or less than or equal to 1 will also ensure the beam 16 focused by the nozzle 2 according to the invention to meet the criteria of a minibeam.

For one given medical facility (or a given medical setup) to another, the divergence of the incident beam 6, the absolute value of the correlation coefficient between a size of the incident beam 6 and the divergence of the incident beam 6, the energy of the incident beam 6 and the arrangement of the medical facility will vary. Hence, the magnetic field gradient generated by the first 10 and/or the second 11 quadrupole are adjusted, through the processing unit, so that the focal length of the second 11 quadrupole is superior or equal to 50 and/or is less than or equal to 240 cm in order for the focused beam 16 to meet the criteria of a minibeam at the focal point of the second 11 quadrupole. The focal point of the first quadrupole 10 and the focal point of the second 11 quadrupole are arranged so that the target 7, or a section plane 18 of the target 7 or an entrance plane 18 of the target 7, is located within the volume extending between the focal point of the first quadrupole 10 and a focal point of the second 11 quadrupole.

Hence, contrary to current setups comprising collimators, which are specifically designed for a given medical facility and/or a patient, the nozzle 2 according to the invention is suitable is suitable to be used in every medical facility only by adjusting the magnetic field gradients of the first 10 and/or of the second 11 quadrupoles.

The invention is not restricted to embodiments described above and numerous adjustments may be made within the scope of the invention.

Thus, in combinable alternatives of previous embodiments:

- the energy of the incident beam 6 is superior or equal to 10 MeV and/or less than or equal to 1000 MeV, preferably less than 500 MeV/nucleon, more preferably comprised between 100 and 230 MeV, and/or
- the focal length of the first quadrupole 10 is superior or equal to 60 and/or is less than or equal to 250 cm, and/or
- the focal length of the first quadrupole 10 and/or the focal length of the second quadrupole 11 is superior or equal to 60 cm, more preferably superior or equal to 70 cm, even more preferably superior or equal to 80 cm and in a preferred manner superior or equal to 90 cm, and/or

- the focal length of the second quadrupole 11 is less than or equal to 180 cm, more preferably less than or equal to 160 cm, even more preferably less than or equal to 150 cm, in a preferred manner less than or equal to 140 cm, in a more preferred manner less than or equal to 130 cm and in a particularly preferred manner less than or equal to 120 cm, and/or

- the focal length of the first quadrupole 10 is less than or equal to 220 cm, more preferably less than or equal to 200 cm, even more preferably less than or equal to 180 cm, in a preferred manner less than or equal to 160 cm, in a more preferred manner less than or equal to 140 cm, in a particularly preferred manner less than or equal to 130 cm, and/or

- the distance d5 is superior or equal to 10 cm and/or less than or equal to 40 cm, more preferably less than or equal to 30 cm, and/or

- the FWHM of the incident beam 6 is less than 50 mm, and/or

- the operational frequencies of the third 12 and fourth 13 magnets is superior or equal to 1 Hz and/or are less than or equal to 200 Hz, and/or

- the focused beam 16 exhibits a FWHM, at a target 7, less than or equal to 0.6 mm, more preferably less than or equal to 0.5 mm.

Moreover, features, alternatives and embodiments of the invention may be associated if they are not mutually exclusive to each other.

**Claims**

1.  Method to generate a minibeam intended to be used for minibeam radiation therapy, said minibeam being generated from an incident beam of charged particles that exhibits:

    • an energy superior or equal to 10 and/or less than or equal to 1000 MeV, and
    • a divergence less than 15 milliradian, and/or
    • an absolute value of a correlation coefficient between a size of the incident beam and the divergence of the incident beam superior or equal to 0.8 and/or less than or equal to 1,

    said method comprising the steps consisting of:

    - focusing the incident beam, through a first quadrupole according to a first direction,
    - focusing the incident beam, through a second quadrupole according to a second direction orthogonal to the first direction,
    - deflecting the incident beam, through a third magnet according to a third direction,
    - deflecting the incident beam through a fourth magnet according to a fourth direction different from the third direction,
    - adjusting a magnetic field gradient generated by the first quadrupole and/or respectively by the second quadrupole so that a focal length of the first quadrupole is superior or equal to 60 and/or is less than or equal to 250 cm and/or respectively a focal length of the second quadrupole is superior or equal to 50 and/or is less than or equal to 200 cm in order for the focused beam to meet the criteria of a minibeam along a volume extending between a focal point of the first quadrupole and a focal point of the second quadrupole.

2.  Method according to claim 1, comprising the step consisting of arranging the beam, the first and second quadrupoles and the third and fourth magnets in a vacuum environment, said vacuum environment extending over a distance higher than 50 cm and lower than 200 cm.

3.  Method according to the preceding claim, wherein a distance from an end of the vacuum environment to the focal point of the second quadrupole is superior to few centimeters and/or is less than or equal to 50 cm.

4.  Method according to any of the preceding claims, wherein a FWHM of the incident beam is less than 50 mm.

5.  Method according to any of the preceding claims, wherein values of magnetic field gradients generated by the first and second quadrupoles, require for the beam focused at the focal point of the second quadrupole to meet the criteria of a minibeam, are superior or equal to 0 and/or are less than or equal to 1,6 $T.cm^{-1}$.

6.  Method according to any of the preceding claims, wherein a distance separating the first quadrupole from the second quadrupole is less than 15 cm, preferably less than 6 cm, more preferably less than or equal to 3 cm.

7. Method according to any of the preceding claims, wherein the incident beam of charged particles exits from a beamline of a medical facility.

8. Method according to any of the preceding claims, wherein the charged particles are ions.

9. Method according to any of the preceding claims, wherein operational frequencies of the third and fourth magnets are superior or equal to 1 Hz and/or are less than or equal to 200 Hz.

10. Method according to any of the preceding claims, wherein a minibeam according to the invention exhibits a horizontal full width at half maximum (hFWHM) less than or equal to 2 mm and a vertical FWHM (vFWHM) equal to or less than the hFWHM of the minibeam.

11. A minibeam scanning nozzle (MSN) for charged particles minibeam Radiation Therapy, said MSN comprising, along a beam path of the charged particles inside the nozzle:

   • a first quadrupole arranged to focus the incident beam according to a first direction,
   • a second quadrupole arranged to focus the incident beam according to a second direction orthogonal to the first direction,
   • a third magnet arranged to deflect the incident beam according to a third direction, and
   • a fourth magnet arranged to deflect the incident beam according to a fourth direction different from the third direction;

   the first quadrupole and/or respectively the second quadrupole being arranged to generate a magnetic field gradient that is adjusted so that a focal length of the first quadrupole is superior or equal to 60 and/or is less than or equal to 250 cm and/or respectively a focal length of the second quadrupole is superior or equal to 50 and/or is less than or equal to 200 cm in order for the focused beam to meet the criteria of a minibeam along a volume extending between a focal point of the first quadrupole and a focal point of the second quadrupole.

12. MSN according to claim 11, comprising a vacuum chamber wherein the first and second quadrupoles and the third and fourth magnets are arranged.

13. MSN according to the preceding claim, wherein a distance between an exit face of the vacuum chamber and the focal point of the second quadrupole is less than 50 cm.

14. MSN according to any of claims 11 to 13, arranged to generate the minibeam from an incident beam of charged particles exiting a beamline of a medical facility, the MSN being intended to be arranged downstream of the beamline in a path of the incident beam of charged particles.

15. MSN according to any of claims 11 to 14, being arranged to carry out the method according to any of claims 1 to 10.

16. Use of the MSN according to any of claims 11 to 15 for implementing the method according to any of claims 1 to 10.

17. System for charged particles minibeam radiation therapy comprising:

   - a beam source arranged to generate an incident beam of charged particles that exhibits:

      • an energy superior or equal to 10 and/or less than or equal to 1000 MeV, and
      • a divergence less than 15 milliradian, and/or
      • an absolute value of a correlation coefficient between a size of the incident beam and the divergence of the incident beam superior or equal to 0.8 and/or less than or equal to 1,

   - a minibeam scanning nozzle (MSN) arranged to generate a minibeam of charged particles comprising, along a beam path of the charged particles inside the nozzle:

      • a first quadrupole arranged to focus the incident beam according to a first direction,
      • a second quadrupole arranged to focus the incident beam according to a second direction orthogonal to the first direction,
- a third magnet arranged to deflect the incident beam according to a third direction, and
- a fourth magnet arranged to deflect the incident beam according to a fourth direction different from the third direction;

the first quadrupole and/or respectively the second quadrupole being arranged to generate a magnetic field gradient that is adjusted so that a focal length of the first quadrupole is superior or equal to 60 and/or is less than or equal to 250 cm and/or respectively a focal length of the second quadrupole is superior or equal to 50 and/or is less than or equal to 200 cm in order for the focused beam to meet the criteria of a minibeam along a volume extending between a focal point of the first quadrupole and a focal point of the second quadrupole.

18. System according to claim 17, wherein the beam source comprises a beamline of a medical facility.

19. System according to claim 17 or 18, wherein the MSN is the MSN according to any of claims 11 to 15.

[Fig. 1]

[Fig. 2]

FIG. 2a

FIG. 2b

FIG. 2c

FIG. 2d

[Fig. 3]

Europäisches
Patentamt

European
Patent Office

Office européen
des brevets

## EUROPEAN SEARCH REPORT

Application Number

EP 19 30 6515

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/213384 A1 (FURUKAWA TAKUJI [JP] ET AL) 26 August 2010 (2010-08-26) <br> * claims; figures * <br> * paragraph [0001] * <br> * paragraph [0022] * <br> * paragraph [0024] * <br> * paragraph [0030] * <br> * paragraph [0032] * <br> * paragraph [0034] * <br> * paragraph [0035] * <br> * paragraph [0044] * <br> * paragraph [0045] * <br> * paragraph [0046] * <br> * paragraph [0049] * <br> * paragraph [0051] * <br> * paragraph [0052] * <br> * paragraph [0053] * <br> * paragraph [0054] * <br> * paragraph [0056] * <br> ----- | 1-19 | INV. <br> A61N5/10 <br> A61B18/26 |
| X | US 2009/032721 A1 (YOSHIDA KATSUHISA [JP]) 5 February 2009 (2009-02-05) <br> * figures * <br> * paragraph [0016] * <br> * paragraph [0024] * <br> ----- | 1-19 | **TECHNICAL FIELDS SEARCHED (IPC)** <br> A61N <br> A61B |
| A | US 2010/187446 A1 (DILMANIAN F AVRAHAM [US] ET AL) 29 July 2010 (2010-07-29) <br> * the whole document * <br> ----- | 1-19 | |

The present search report has been drawn up for all claims

| Place of search | Date of completion of the search | Examiner |
|---|---|---|
| Munich | 31 March 2020 | Rodríguez Cosío, J |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

**ANNEX TO THE EUROPEAN SEARCH REPORT
ON EUROPEAN PATENT APPLICATION NO.**

EP 19 30 6515

This annex lists the patent family members relating to the patent documents cited in the above-mentioned European search report.
The members are as contained in the European Patent Office EDP file on
The European Patent Office is in no way liable for these particulars which are merely given for the purpose of information.

31-03-2020

| Patent document cited in search report | | Publication date | Patent family member(s) | | Publication date |
|---|---|---|---|---|---|
| US 2010213384 | A1 | 26-08-2010 | JP | 4474549 B2 | 09-06-2010 |
| | | | JP | 2006346120 A | 28-12-2006 |
| | | | US | 2010213384 A1 | 26-08-2010 |
| | | | WO | 2006134677 A1 | 21-12-2006 |
| US 2009032721 | A1 | 05-02-2009 | CN | 101002978 A | 25-07-2007 |
| | | | JP | 4591356 B2 | 01-12-2010 |
| | | | JP | 2007185423 A | 26-07-2007 |
| | | | US | 2007164227 A1 | 19-07-2007 |
| | | | US | 2009032721 A1 | 05-02-2009 |
| US 2010187446 | A1 | 29-07-2010 | NONE | | |

EPO FORM P0459

For more details about this annex : see Official Journal of the European Patent Office, No. 12/82